# EUROPÄISCHE PATENTSCHRIFT

(11) **EP 1 177 273 B1**
(45) Veröffentlichungstag und Bekanntmachung des Hinweises auf die Patenterteilung: **15.06.2005**
(21) Anmeldenummer: 00934972.1
(22) Anmeldetag: 28.04.2000
(51) Int. Cl.: C11D 3/20, A61K 7/48

(54) **HOCHKONZENTRIERT FLIESSFÄHIGE PERLGLANZKONZENTRATE**
HIGHLY CONCENTRATED FREE-FLOWING PEARLY LUSTRE CONCENTRATES
CONCENTRATS FLUIDES, D'UN LUSTRE NACRE, HAUTEMENT CONCENTRES

(30) Priorität: 07.05.1999 DE 19921186
(43) Veröffentlichungstag der Anmeldung: 06.02.2002
(73) Patentinhaber: Cognis Deutschland GmbH & Co. KG, 40589 Düsseldorf (DE)
(72) Erfinder: NIEENDICK, Claus, 47807 Krefeld (DE); SCHMID, Karl-Heinz, 40822 Mettmann (DE); NALBORCZYK, Mirella, 40231 Düsseldorf (DE)
(86) Internationale Anmeldenummer: PCT/EP2000/003854
(87) Internationale Veröffentlichungsnummer: WO 2000/068350

(56) Entgegenhaltungen:
- EP-A- 0 300 379
- EP-A- 0 569 028
- DE-A- 4 103 551
- DE-A- 19 621 681
- DE-C- 19 801 231

## Beschreibung

### Gebiet der Erfindung

Die Erfindung betrifft hochkonzentrierte Perlglanzkonzentrate mit einem Gehalt an Perlglanzwachsen, speziellen Emulgatormischungen und gegebenenfalls Polyolen, ein Verfahren zu ihrer Herstellung sowie die Verwendung von bestimmten Co-Emulgatoren als Viskositätsregulatoren zur Herstellung von Periglanzkonzentraten mit hohem Aktivsubstanzgehalt.

### Stand der Technik

Der weich schimmernde Glanz von Perien hat auf den Menschen schon seit Jahrtausenden eine besondere Faszination ausgeübt. Es ist daher kein Wunder, daß die Hersteller von kosmetischen Zubereitungen versuchen, ihren Produkten ein attraktives, wertvolles und gehaltvolles Erscheinungsbild zu verleihen. Der erste seit dem Mittelalter in der Kosmetik eingesetzte Perlglanz war eine perlglänzende Paste aus natürlichen Fischschuppen. Zu Anfang dieses Jahrhunderts entdeckte man, daß Wismutoxidchloride ebenfalls in der Lage sind, Perlglanz zu erzeugen. Für die moderne Kosmetik sind hingegen Perlglanzwachse, insbesondere vom Typ der Glycolmono- und -difettsäureester von Bedeutung, die überwiegend zur Erzeugung von Perlglanz in Haarshampoos und Duschgelen eingesetzt werden. Eine Übersicht zu modernen, perlglänzenden Formulierungen findet sich von A.Ansmann und R.Kawa in **Parf.Kosm.** **75****, 578 (1994).**

Der Stand der Technik kennt eine Vielzahl von Formulierungen, die oberflächenaktiven Mitteln den gewünschten Perlglanz verleihen. So sind beispielsweise aus den beiden deutschen Patentanmeldungen **DE 3843572 A1** und **DE 4103551 A1** (Henkel) Perlglanzkonzentrate in Form fließfähiger wäßriger Dispersionen bekannt, die 15 bis 40 Gew.-% perlglänzender Komponenten, 5 bis 55 Gew.-% Emulgatoren und 0,1 bis 5 bzw. 15 bis 40 Gew.-% Polyole enthalten. Bei den Perlglanzwachsen handelt es sich um acylierte Polyalkylenglycole, Monoalkanolamide, lineare, gesättigte Fettsäuren oder Ketosulfone. In den beiden Europäischen Patentschriften **EP 0181773 B1** und **EP 0285389 B1** (Procter & Gamble) werden Shampoozusammensetzungen vorgeschlagen, die Tenside, nicht-flüchtige Silicone und Perlglanzwachse enthalten. Gegenstand der Europäischen Patentanmeldung **EP 0205922 A2** (Henkel) sind fließfähige Perlglanzkonzentrate, die 5 bis 15 Gew.-% acylierte Polyglycole, 1 bis 6 Gew.-% Fettsäuremonoethanolamide und 1 bis 5 Gew.-% nichtionische Emulgatoren enthalten. Gemäß der Lehre der Europäischen Patentschrift **EP 0569843 B1** (Hoechst) lassen sich nichtionische, fließfähige Perlglanzdispersionen auch erhalten, indem man Mischungen von 5 bis 30 Gew.-% acylierten Polyglycolen und 0,1 bis 20 Gew.-% ausgewählten nichtionischen Tensiden herstellt. Aus der Europäischen Patentanmeldung **EP 0581193 A2** (Hoechst) sind ferner fließfähige, konservierungsmittelfreie Perlglanzdispersionen bekannt, die acylierte Polyglycolether, Betaine, Aniontenside und Glycerin enthalten. Schließlich wird in der Europäischen Patentanmeldung **EP 0684302 A1** (Th.Goldschmidt) die Verwendung von Polyglycerinestern als Kristallisationshilfsmittel für die Herstellung von Perlglanzkonzentraten vorgeschlagen.

Trotz der Vielzahl von Mitteln besteht im Markt ein ständiges Bedürfnis nach neuen Perlglanzkonzentraten, die auch bei extrem hohen Aktivsubstanzgehalten noch fließfähig sind, über ausgezeichnete anwendungstechnische Eigenschaften verfügen, die Mitverwendung kritischer Inhaltsstoffe wie beispielsweise von Siliconen zulassen, ohne daß die Stabilität der Formulierungen beeinträchtigt wird, und in den Endformulierungen weitere positive Effekte bewirken. Die Aufgabe der vorliegenden Erfindung hat somit darin bestanden, neue Perlglanzkonzentrate mit dem geschilderten komplexen Anforderungsprofil zur Verfügung zu steilen.

### Beschreibung der Erfindung

Gegenstand der Erfindung sind hochkonzentriert fließfähige Perlglanzkonzentrate, enthaltend
(a) 25 bis 45, vorzugsweise 30 bis 40 Gew.-% Perlglanzwachse,
(b) 25 bis 40, vorzugsweise 30 bis 35 Gew.-% nichtionische, amphotere, zwitterionische und/oder kationische Emulgatoren und
(c) 0,5 bis 15, vorzugsweise 1 bis 10 Gew.-% Polyolester
mit den Maßgaben, daß sich die Mengenangaben mit Wasser und gegebenenfalls weiteren Hilfs- und Zusatzstoffen zu 100 Gew.-% ergänzen und die Summe der Komponenten (a), (b) und (c) mindestens 55, vorzugsweise mindestens 60 und insbesondere 65 bis 70 Gew.-% beträgt.

Überraschenderweise wurde gefunden, daß die Mitverwendung von Polyolestern die Herstellung von Perlglanzkonzentraten erlaubt, die einen gegenüber bekannten Zubereitungen nach dem Stand der Technik deutlich erhöhten Aktivsubstanzgehalt (entsprechend der Summe aus Perlglanzwachsen und Emulgatoren/Co-Emulgatoren) aufweisen und dennoch bei Raumtemperatur fließfähig sind. Die neuen Perlglanzkonzentrate sind besonders feinteilig und verleihen wäßrigen tensidischen Zubereitungen einen besonders dichten und brillanten Perlglanz. Werden sie zur Herstellung von Haarbehandlungsmitteln eingesetzt, verbessern sie zudem Glanz und Weichgriff des gewaschenen Haares. Die Einarbeitung von Siliconverbindungen ist problemlos möglich.

### Perlglanzwachse

Als Perlglanzwachse kommen beispielsweise in Frage: Alkylenglycolester, Fettsäurealkanolamide, Partialglyceride, Ester von mehrwertigen, gegebenenfalls hydroxysubstituierten Carbonsäuren, Fettalkohole, Fettsäuren, Fettketone, Fettaldehyde, Fettether, Fettcarbonate, Ringöffnungsprodukte von Olefinepoxiden sowie deren Mischungen.

Bei den **Alkylenglycolestern,** die die Komponente (a1) bilden, handelt es sich üblicherweise um Mono- und/oder Diester von Alkylenglycolen, die der Formel **(III)** folgen,

**R**^{**5**}**CO(OA)**_{**n**}**OR**^{**6**} **(III)**

in der R⁵CO für einen linearen oder verzweigten, gesättigten oder ungesättigten Acylrest mit 6 bis 22 Kohlenstoffatomen, R⁶ für Wasserstoff oder R⁵CO und A für einen linearen oder verzweigten Alkylenrest mit 2 bis 4 Kohlenstoffatomen und n für Zahlen von 1 bis 5 steht. Typische Beispiele sind Mono- und/oder Diester von Ethylenglycol, Propylenglycol, Diethylenglycol, Dipropylenglycol, Triethylenglycol oder Tetraethylenglycol mit Fettsäuren mit 6 bis 22, vorzugsweise 12 bis 18 Kohlenstoffatomen als da sind: Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Besonders bevorzugt ist der Einsatz von Ethylenglycolmono- und/oder -distearat.

**Fettsäurealkanolamide,** die als Perlglanzwachse der Gruppe (a2) in Frage kommen, folgen der Formel **(IV),**

**R**^{**7**}**CO-NR**^{**8**}**-B-OH (IV)**

in der R⁷CO für einen linearen oder verzweigten, gesättigten oder ungesättigten Acylrest mit 6 bis 22 Kohlenstoffatomen, R⁸ für Wasserstoff oder einen gegebenenfalls hydroxysubstituierten Alkylrest mit 1 bis 4 Kohlenstoffatomen und B für eine lineare oder verzweigte Alkylengruppe mit 1 bis 4 Kohlenstoffatomen steht. Typische Beispiele sind Kondensationsprodukte von Ethanolamin, Methylethanolamin, Diethanolamin, Propanolamin, Methylpropanolamin und Dipropanolamin sowie deren Mischungen mit Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Besonders bevorzugt ist der Einsatz von Stearinsäureethanolamid.

**Partialglyceride,** die über Perlglanzeigenschaften verfügen und die Komponente (a3) bilden, stellen Mono und/oder Diester des Glycerins mit linearen, gesättigten Fettsäuren, nämlich beispielsweise Capronsäure, Caprylsäure, Caprinsäure, Laurinsäure, Myristinsäure, Palmitinsäure, Palmoleinsäure, Stearinsäure, Behensäure sowie deren technische Mischungen dar. Sie folgen der Formel **(V)**, 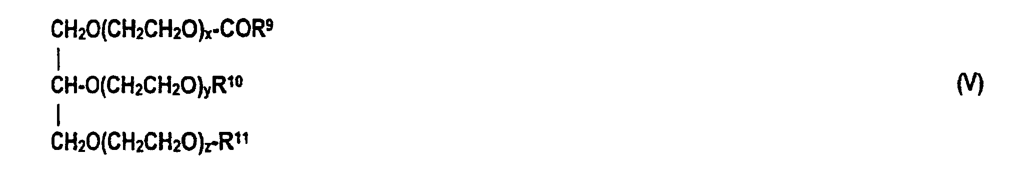 in der R⁹CO für einen linearen, gesättigten Acylrest mit 6 bis 22 Kohlenstoffatomen, R¹⁰ und R¹¹ unabhängig voneinander für Wasserstoff oder R⁹CO, x, y und z in Summe für 0 oder für Zahlen von 1 bis 30 und X für ein Alkali- oder Erdalkalimetall mit der Maßgabe steht, daß mindestens einer der beiden Reste R¹⁰ und R¹¹ Wasserstoff darstellt. Typische Beispiele sind Laurinsäuremonoglycerid, Laurinsäurediglycerid, Kokosfettsäuremonoglycerid, Kokosfettsäuretriglycerid, Palmitinsäuremonoglycerid, Palmitinsäuretriglycerid, Stearinsäuremonoglycerid, Stearinsäurediglycerid, Talgfettsäuremonoglycerid, Talgfettsäurediglycerid, Behensäuremonoglycerid, Behensäurediglycerid sowie deren technische Gemische, die untergeordnet aus dem Herstellungsprozeß noch geringe Mengen an Triglycerid enthalten können.

Als Perlglanzwachse, die die Komponente (a4) bilden, kommen weiterhin **Ester von mehrwertigen, gegebenenfalls hydroxysubstituierten Carbonsäuren** mit Fettalkoholen mit 6 bis 22 Kohlenstoffatomen in Frage. Als Säurekomponente dieser Ester kommen beispielsweise Malonsäure, Maleinsäure, Fumarsäure, Adipinsäure, Sebacinsäure, Azelainsäure, Dodecandisäure, Phthalsäure, Isophthalsäure und insbesondere Bernsteinsäure sowie Äpfelsäure, Citronensäure und insbesondere Weinsäure und deren Mischungen in Betracht. Die Fettalkohole enthalten 6 bis 22, vorzugsweise 12 bis 18 und insbesondere 16 bis 18 Kohlenstoffatome in der Alkylkette. Typische Beispiele sind Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen. Die Ester können als Voll oder Partialester vorliegen, vorzugsweise werden Mono- und vor allem Diester der Carbon- bzw. Hydroxycarbonsäuren eingesetzt. Typische Beispiele sind Bernsteinsäuremono- und - dilaurylester, Bernsteinsäuremono- und -dicetearlyester, Bernsteinsäuremono- und -distearylester, Weinsäuremono- und -dilaurylester, Weinsäuremono- und dikokosalkylester, Weinsäuremono- und - dicetearylester, Citronensäuremono-, -di- und -trilaurylester, Citronensäuremono-, -di- und - trikokosalkylester sowie Citronensäuremono-, -di- und -tricetearylester.

Als weitere Gruppe von Perlglanzwachsen (a5) können **Fettalkohole** und/oder **Fettsäuren** eingesetzt werden, die der Formel **(VI)** folgen,

**R**^{**12**}**OH (VI)**

in der R¹² für einen linearen, gegebenenfalls hydroxysubstituierten Alkylrest und/oder Acylrest mit 16 bis 48, vorzugsweise 18 bis 36 Kohlenstoffatomen steht. Typische Beispiele für geeignete Alkohole sind Cetearylalkohol, Hydroxystearylalkohol, Behenylalkohol sowie Oxidationsprodukte langkettiger Paraffine, als Beispiele für Säuren kommen Stearinsäure, Hydroxystearinsäure sowie insbesondere Behensäure in Frage, letztere vorzugsweise in einer Reinheit oberhalb von 90 Gew.-%.

Fettketone, die als Komponente (a6) in Betracht kommen, folgen vorzugsweise der Formel **(VII),**

**R**^{**13**}**-CO-R**^{**14**} **(VII)**

in der R¹³ und R¹⁴ unabhängig voneinander für Alkyl- und/oder Alkenylreste mit 1 bis 22 Kohlenstoffatomen stehen, mit der Maßgabe, daß sie in Summe mindestens 24 und vorzugsweise 32 bis 48 Kohlenstoffatome aufweisen. Die Ketone können nach Verfahren des Stands der Technik hergestellt werden, beispielsweise durch Pyrolyse der entsprechenden Fettsäure-Magnesiumsalze. Die Ketone können symmetrisch oder unsymmetrisch aufgebaut sein, vorzugsweise unterscheiden sich die beiden Reste R¹³ und R¹⁴ aber nur um ein Kohlenstoffatom und leiten sich von Fettsäuren mit 16 bis 22 Kohlenstoffatomen ab. Dabei zeichnet sich Stearon durch besonders vorteilhafte Perlglanzeigenschaften aus.

Als Perlglanzwachse geeignete **Fettaldehyde** (a7) entsprechen vorzugsweise der Formel **(VIII),**

**R**^{**15**}**COH (VIII)**

in der R¹⁵CO für einen linearen oder verzweigten Acylrest mit 24 bis 48, vorzugsweise 28 bis 32 Kohlenstoffatomen steht.

Als Perlglanzwachse (a8) kommen ferner **Fettether** vorzugsweise der Formel **(IX)** in Frage,

**R**^{**16**}**-O-R**^{**17**} **(IX)**

in der R¹⁶ und R¹⁷ unabhängig voneinander für Alkyl- und/oder Alkenylreste mit 1 bis 22 Kohlenstoffatomen stehen, mit der Maßgabe, daß sie in Summe mindestens 24 und vorzugsweise 32 bis 48 Kohlenstoffatome aufweisen. Fettether der genannten Art werden üblicherweise durch saure Kondensation der entsprechenden Fettalkohole hergestellt. Fettether mit besonders vorteilhaften Perlglanzeigenschaften werden durch Kondensation von Fettalkoholen mit 16 bis 22 Kohlenstoffatomen, wie beispielsweise Cetylalkohol, Cetearylalkohol, Stearylalkohol, Isostearylalkohol, Oleylalkohol, Behenylalkohol und/oder Erucylalkohol erhalten.

Als Komponente (a9) kommen weiterhin **Fettcarbonate** vorzugsweise der Formel **(X)** in Betracht,

**R**^{**18**}**O-CO-OR**^{**19**} **(X)**

in der R¹⁸ und R¹⁹ unabhängig voneinander für Alkyl- und/oder Alkenylreste mit 1 bis 22 Kohlenstoffatomen stehen, mit der Maßgabe, daß sie in Summe mindestens 24 und vorzugsweise 32 bis 48 Kohlenstoffatome aufweisen. Die Stoffe werden erhalten, indem man beispielsweise Dimethyl- oder Diethylcarbonat mit den entsprechenden Fettalkoholen in an sich bekannter Weise umestert. Demzufolge können die Fettcarbonate symmetrisch oder unsymmetrisch aufgebaut sein. Vorzugsweise werden jedoch Carbonate eingesetzt, in denen R¹⁸ und R¹⁹ gleich sind und für Alkylreste mit 16 bis 22 Kohlenstoffatomen stehen. Besonders bevorzugt sind Umesterungsprodukte von Dimethyl- bzw. Diethylcarbonat mit Cetylalkohol, Cetearylalkohol, Stearylalkohol, Isostearylalkohol, Oleylalkohol, Behenylalkohol und/ oder Erucylalkohol in Form ihrer Mono- und Diester bzw. deren technischen Mischungen.

Bei den **Epoxidringöffnungsprodukten,** die schließlich die Gruppe (a10) bilden, handelt es sich um bekannte Stoffe, die üblicherweise durch säurekatalysierte Umsetzung von endständigen oder innenständigen Olefinepoxiden mit aliphatischen Alkoholen hergestellt werden. Die Reaktionsprodukte folgen vorzugsweise der Formel **(XI),** 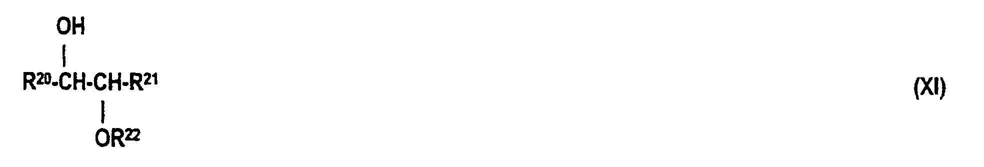 in der R²⁰ und R²¹ für Wasserstoff oder einen Alkylrest mit 10 bis 20 Kohlenstoffatomen steht, mit der Maßgabe, daß die Summe der Kohlenstoffatome von R²⁰ und R²¹ im Bereich von 10 bis 20 liegt und R²² für einen Alkyl- und/oder Alkenylrest mit 12 bis 22 Kohlenstoffatomen und/oder den Rest eines Polyols mit 2 bis 15 Kohlenstoffatomen und 2 bis 10 Hydroxylgruppen steht. Typische Beispiele sind Ringöffnungsprodukte von α-Dodecenepoxid, α-Hexadecenepoxid, α-Octadecenepoxid, α-Eicosenepoxid, α-Docosenepoxid, i-Dodecenepoxid, i-Hexadecenepoxid, i-Octadecenepoxid, i-Eicosenepoxid und/ oder i-Docosenepoxid mit Laurylalkohol, Kokosfettalkohol, Myristylalkohol, Cetylalkohol, Cetearylalkohol, Stearylalkohol, Isostearylalkohol, Oleylalkohol, Elaidylalkohol, Petroselinylalkohol, Linolylalkohol, Linolenylalkohol, Behenylalkohol und/oder Erucylalkohol. Vorzugsweise werden Ringöffnungsprodukte von Hexa- und/oder Octadecenepoxiden mit Fettalkoholen mit 16 bis 18 Kohlenstoffatomen eingesetzt. Werden anstelle der Fettalkohole Polyole für die Ringöffnung eingesetzt, so handelt es sich beispielsweise um folgende Stoffe: Glycerin; Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton; technische Oligoglyceringemische mit einem Eigenkondensationsgrad von 1,5 bis 10 wie etwa technische Diglyceringemische mit einem Diglyceringehalt von 40 bis 50 Gew.-%; Methyolverbindungen, wie insbesondere Trimethylolethan, Trimethylolpropan, Trimethylolbutan, Pentaerythrit und Dipentaerythrit; Niedrigalkylglucoside, insbesondere solche mit 1 bis 8 Kohlenstoffen im Alkylrest, wie beispielsweise Methyl- und Butylglucosid; Zuckeralkohole mit 5 bis 12 Kohlenstoffatomen, wie beispielsweise Sorbit oder Mannit, Zucker mit 5 bis 12 Kohlenstoffatomen, wie beispielsweise Glucose oder Saccharose; Aminozucker, wie beispielsweise Glucamin.

### Emulgatoren

Als Emulgatoren (Komponente b) kommen beispielsweise **nichtionogene Tenside** aus mindestens einer der folgenden Gruppen in Frage:
(b1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
(b2) Alkylmono- und -oligoglycoside mit 8 bis 22 Kohlenstoffatomen im Alkylrest und deren ethoxylierte Analoga;
(b3) Anlagerungsprodukte von 1 bis 15 Mol Ethylenoxid an Ricinusöl und/oder gehärtetes Ricinusöl;
(b4) Anlagerungsprodukte von 15 bis 60 Mol Ethylenoxid an Ricinusöl und/oder gehärtetes Ricinusöl;
(b5) Mono-, Di- und Trialkylphosphate sowie Mono-, Di- und/oder Tri-PEG-alkylphosphate und deren Salze;
(b6) Wollwachsalkohole;
(b7) Polysiloxan-Polyalkyl-Polyether-Copolymere bzw. entsprechende Derivate;
(b8) Polyalkylenglycole sowie
(b9) Glycerincarbonat.

Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE 2024051 PS** als Rückfettungsmittel für kosmetische Zubereitungen bekannt. C_{8/18}-Alkylmono- und -oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Weiterhin können als Emulgatoren **zwitterionische Tenside** verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin.

Schließlich kommen auch **Kationtenside** als Emulgatoren in Betracht, wobei solche vom Typ der Esterquats, vorzugsweise methylquaternierte Difettsäuretriethanolaminester-Salze, besonders bevorzugt sind.

### Polyolester

Polyolester, die als Co-Emulgatoren die Komponente (c) bilden, können ausgewählt sein aus den folgenden Gruppen von Verbindungen:
(c1) Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
(c2) Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
(c3) Mischester aus Pentaerythrit, Fettsäuren, Citronensäure und Fettalkohol gemäß **DE 1165574 PS** und/oder Mischester von Fettsäuren mit 6 bis 22 Kohlenstoffatomen, Methylglucose und Polyolen, vorzugsweise Glycerin oder Polyglycerin.

Typische Beispiele für geeignete **Partialglyceride** sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

Als **Sorbitanester** kommen Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

Typische Beispiele für geeignete Polyglycerinester sind Polyglyceryl-2 Dipolyhydroxystearate (Dehymuls® PGPH), Polyglycerin-3-Diisostearate (Lameform® TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische.

Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Sterainsäure, Ölsäure, Behensäure und dergleichen.

### Polyole

In einer bevorzugten Ausführungsform der Erfindung können die Konzentrate zur Viskositätsverminderung als fakultative Komponente (d) weiterhin Polyole enthalten. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol.

Die Einsatzmenge der Polyole liegt typischerweise im Bereich von 0,1 bis 15 und vorzugsweise 0,5 bis 5 Gew.-%. Werden höhere Polyolmengen, vorzugsweise Glycerin oder Ethylenglycol eingesetzt, werden die Konzentrate gleichzeitig gegen mikrobiellen Befall stabilisiert.

### Herstellverfahren

In einer bevorzugten Ausführungsform, die ebenfalls Gegenstand der Erfindung ist, erfolgt die Herstellung der Perlglanzkonzentrate, indem man eine Mischung aus den Komponenten (a), (b), (c) und gegebenenfalls (d) herstellt, auf eine Temperatur erwärmt, die 1 bis 30 °C oberhalb des Schmelzpunktes der Mischung liegt, mit der erforderlichen Menge Wasser etwa der gleichen Temperatur mischt und anschließend auf Raumtemperatur abkühlt. Femer ist es möglich, eine konzentrierte wäßrige (Anion)-Tensidpaste vorzulegen, das Perlglanzwachs in der Wärme einzurühren und die Mischung anschließend mit weiterem Wasser auf die gewünschte Konzentration zu verdünnen oder das Vermischen in Gegenwart polymerer hydrophiler Verdickungsmittel, wie etwa Hydroxypropylcellulosen, Xanthan Gum oder Polymeren vom Carbomer-Typ durchzuführen.

### Gewerbliche Anwendbarkeit

Die erfindungsgemäßen Perlglanzkonzentrate eignen sich zur Einstellung einer Trübung in oberflächenaktiven Zubereitungen wie beispielsweise Haarshampoos oder manuellen Geschirrspülmitteln. Zur Herstellung getrübter und perlglänzender flüssiger, wäßriger Zubereitungen wasserlöslicher grenzflächenaktiver Stoffe, werden den klaren wäßrigen Zubereitungen üblicherweise bei 0 bis 40 °C die Perlglanzkonzentrate in einer Menge von 0,5 bis 40, vorzugsweise 1 bis 20 Gew.-% der Zubereitung zusetzt und unter Rühren darin verteilt. Ein weiterer Gegenstand der Erfindung besteht schließlich in der Verwendung von Polyolestern als Viskositätsregulatoren zur Herstellung von Perlglanzkonzentraten mit Aktivsubstanzgehalten von mindestens 55 Gew.-%.

### Kosmetische und/oder pharmazeutische Zubereitungen

Die erfindungsgemäßen Perlglanzkonzentrate können zur Herstellung von kosmetischen und/oder pharmazeutischen Zubereitungen, wie beispielsweise Haarshampoos, Haarlotionen, Schaumbäder, Duschbäder, Cremes, Gele, Lotionen, alkoholische und wäßrig/alkoholische Lösungen, Emulsionen, Wachs/ Fett-Massen, Stiftpräparaten, Pudern oder Salben dienen. Diese Mittel können ferner als weitere Hilfs- und Zusatzstoffe milde Tenside, Ölkörper, Überfettungsmittel, Konsistenzgeber, Verdickungsmittel, Polymere, Siliconverbindungen, Fette, Wachse, Stabilisatoren, biogene Wirkstoffe, Deodorantien, Antitranspirantien, Antischuppenmittel, Filmbildner, Quellmittel, UV-Lichtschutzfaktoren, Antioxidantien, Hydrotrope, Konservierungsmittel, Insektenrepellentien, Selbstbräuner, Solubilisatoren, Parfümöle, Farbstoffe und dergleichen enthalten.

Typische Beispiele für geeignete milde, d.h. besonders hautverträgliche **Tenside** sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine und/oder Proteinfettsäurekondensate, letztere vorzugsweise auf Basis von Weizenproteinen.

Als **Ölkörper** kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von Hydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, Guerbetcarbonate, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, Ringöffnungsprodukte von epoxidierten Fettsäureestern mit Polyolen, Siliconöle und/oder aliphatische bzw. naphthenische Kohlenwasserstoffe, wie z.B. wie Squalan, Squalen oder Dialkylcyclohexane in Betracht.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als **Konsistenzgeber** kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten.

Geeignete **Verdickungsmittel** sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, (z.B. Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quaternierte Kollagenpolypeptide, wie beispielsweise Lauryldimonium hydroxypropyl hydrolyzed collagen (Lamequat®L/Grünau), quaternierte Weizenpolypeptide, Polyethylenimin, kationische Siliconpolymere, wie z.B. Amidomethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide, wie z.B. beschrieben in der **FR 2252840 A** sowie deren vemetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als **anionische, zwitterionische, amphotere und nichtionische Polymere** kommen beispielsweise Vinylacetat/Crotonsäure-Copolymere, Vinylpyrrolidon/Vinylacrylat-Copolymere, Vinylacetat/Butylmaleat/Isobomylacrylat-Copolymere, Methylvinylether/Maleinsäureanhydrid-Copolymere und deren Ester, unvemetzte und mit Polyolen vemetzte Polyacrylsäuren, Acrylamidopropyltrimethylammoniumchlorid/Acrylat-Copolymere, Octylacrylamid/Methylmethacrylat/tert.Butylaminoethylmethacrylat/2-Hydroxyproylmethacrylat-Copolymere, Polyvinylpyrrolidon, Vinylpyrrolidon/Vinylacetat-Copolymere, Vinylpyrrolidon/Dimethylaminoethylmethacrylat/Vinylcaprolactam-Terpolymere sowie gegebenenfalls derivatisierte Celluloseether und Silicone in Frage.

Geeignete **Siliconverbindungen** sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt. Eine detaillierte Übersicht über geeignete flüchtige Silicone findet sich zudem von Todd et al. in **Cosm.Toil.** **91****, 27 (1976).**

Typische Beispiele für **Fette** sind Glyceride, als **Wachse** kommen u.a. natürliche Wachse, wie z.B. Candelillawachs, Camaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage.

Als **Stabilisatoren** können Metallsalze von Fettsäuren, wie z.B. Magnesium-, Aluminium- und/oder Zinkstearat bzw. -ricinoleat eingesetzt werden.

Unter **biogenen Wirkstoffen** sind beispielsweise Tocopherol, Tocopherolacetat, Tocopherolpalmitat, Ascorbinsäure, Desoxyribonucleinsäure, Retinol, Bisabolol, Allantoin, Phytantriol, Panthenol, AHA-Säuren, Aminosäuren, Ceramide, Pseudoceramide, essentielle Öle, Pflanzenextrakte und Vitaminkomplexe zu verstehen.

Kosmetische **Deodorantien** (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren.

Als **keimhemmende Mittel** sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4 dichlorphenyl)hamstoff, 2,4,4'-Trichlor-2'-hydroxydiphenylether (Triclosan), 4-Chlor-3,5-dimethylphenol, 2,2'-Methylen-bis(6-brom-4-chlorphenol), 3-Methyl-4-(1-methylethyl)phenol, 2-Benzyl-4-chlorphenol, 3-(4-Chlorphenoxy)-1,2-propandiol, 3-Iod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonolaurat (GML), Diglycerinmonocaprinat (DMC), Salicylsäure-N-alkylamide wie z. B. Salicylsäure-n-octylamid oder Salicylsäure-n-decylamid.

Als **Enzyminhibitoren** sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT, Henkel KGaA, Düsseldorf/FRG). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbnonsäuren und deren Ester wie beispielsweise Citronensäure, Äpfelsäure, Weinsäure oder Weinsäurediethylester, sowie Zinkglycinat.

Als **Geruchsabsorber** eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzem, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert.-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evemyl, Iraldein gamma, Phenylessigsäure, Geranylacetat, Benzylacetat, Rosenoxid, Romilat, Irotyl und Floramat allein oder in Mischungen, eingesetzt.

**Antitranspirantien** (Antiperspirantien) reduzieren durch Beeinflussung der Aktivität der ekkrinen Schweißdrüsen die Schweißbildung, und wirken somit Achselnässe und Körpergeruch entgegen. Wässrige oder wasserfreie Formulierungen von Antitranspirantien enthalten typischerweise folgende Inhaltsstoffe:
(a) adstringierende Wirkstoffe,
(b) Ölkomponenten,
(c) nichtionische Emulgatoren,
(d) Coemulgatoren,
(e) Konsistenzgeber,
(f) Hilfsstoffe wie z. B. Verdicker oder Komplexierungsmittel und/oder
(g) nichtwässrige Lösungsmittel wie z. B. Ethanol, Propylenglykol und/oder Glycerin.

Als adstringierende Antitranspirant-Wirkstoffe eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichlorohydrat, Aluminium-Zirkonium-tetrachlorohydrat, Aluminium-Zirkoniumpentachlorohydrat und deren Komplexverbindungen z. B. mit Aminosäuren wie Glycin. Daneben können in Antitranspirantien übliche öllösliche und wasserlösliche Hilfsmittel in geringeren Mengen enthalten sein. Solche öllöslichen Hilfsmittel können z.B. sein:
- entzündungshemmende, hautschützende oder wohlriechende ätherische Öle,
- synthetische hautschützende Wirkstoffe und/oder
- öllösliche Parfümöle.

Übliche wasserlösliche Zusätze sind z.B. Konservierungsmittel, wasserlösliche Duftstoffe, pH-Wert-Stellmittel, z.B. Puffergemische, wasserlösliche Verdickungsmittel, z.B. wasserlösliche natürliche oder synthetische Polymere wie z.B. Xanthan-Gum, Hydroxyethylcellulose, Polyvinylpyrrolidon oder hochmolekulare Polyethylenoxide.

Als **Antischuppenmittel** können Climbazol, Octopirox und Zinkpyrethion eingesetzt werden.

Gebräuchliche **Filmbildner** sind beispielsweise Chitosan, mikrokristallines Chitosan, quatemiertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

Als **Quellmittel** für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in **Cosm.Toil.** **108****, 95 (1993)** entnommen werden.

Unter **UV-Lichtschutzfaktoren** sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
- 3-Benzylidencampher bzw. 3-Benzylidennorcampher und dessen Derivate, z.B. 3-(4-Methylbenzyliden)campher wie in der **EP 0693471 B1** beschrieben;
- 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
- Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
- Ester der Salicylsäure, vorzugsweise Salicylsäure-2-ethylhexylester, Salicylsäure-4-isopropylbenzylester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexylester,
- Triazinderivate, wie z.B. 2,4,6-Trianilino-(p-carbo-2'-ethyl-1'-hexyloxy)-1,3,5-triazin und Octyl Triazon, wie in der **EP 0818450 A1** beschrieben oder Dioctyl Butamido Triazone (Uvasorb® HEB);
- Propan-1,3-dione, wie z.B. 1-(4-tert.Butylphenyl)-3-(4'methoxyphenyl)propan-1,3-dion;
- Ketotricyclo(5.2.1.0)decan-Derivate, wie in der **EP 0694521 B1** beschrieben.

Als wasserlösliche Substanzen kommen in Frage:
- 2-Phenylbenzimidazol-5-sulfonsäure und deren Alkali-, Erdalkali-, Ammonium-, Alkylammonium-, Alkanolammonium- und Glucammoniumsalze;
- Sulfonsäurederivate von Benzophenonen, vorzugsweise 2-Hydroxy-4-methoxybenzophenon-5-sulfonsäure und ihre Salze;
- Sulfonsäurederivate des 3-Benzylidencamphers, wie z.B. 4-(2-Oxo-3-bomylidenmethyl)benzolsulfonsäure und 2-Methyl-5-(2-oxo-3-bomyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol 1789), 1-Phenyl-3-(4'-isopropylphenyl)-propan-1,3-dion sowie Enaminverbindungen, wie beschrieben in der **DE 19712033 A1** (BASF). Die UV-A und UV-B-Filter können selbstverständlich auch in Mischungen eingesetzt werden. Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P.Finkel in **SÖFW-Journal 122, 543 (1996)** zu entnehmen.

Neben den beiden vorgenannten Gruppen primärer Lichtschutzstoffe können auch sekundäre Lichtschutzmittel vom Typ der **Antioxidantien** eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Zur Verbesserung des Fließverhaltens können ferner Hydrotrope, wie beispielsweise Ethanol oder Isopropylalkohol.

Als **Konservierungsmittel** eignen sich beispielsweise Phenoxyethanol, Formaldehydlösung, Parabene, Pentandiol oder Sorbinsäure sowie die in Anlage 6, Teil A und B der Kosmetikverordnung aufgeführten weiteren Stoffklassen. Als **Insekten-Repellentien** kommen N,N-Diethyl-m-toluamid, 1,2-Pentandiol oder Ethyl Butylacetylaminopropionate in Frage, als Selbstbräuner eignet sich Dihydroxyaceton.

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzem (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe.

Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, ∝-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citroneliol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt - oder Heißprozesse erfolgen; vorzugsweise arbeitet man nach der Phaseninversionstemperatur-Methode.

### Beispiele

Die erfindungsgemäßen Periglanzkonzentrate 1 bis 8 sowie die Vergleichsmischung V1 wurden 14 Tage bei 40°C gelagert und die Viskosität nach der Brookfield-Methode in einem RVT-Viskosimeter (23°C, 10 Upm, Spindel 5) bestimmt. Anschließend wurden wäßrige Haarshampooformulierungen durch Vermischen der Einsatzstoffe bei 20°C zubereitet, die jeweils 2 g der Perlglanzkonzentrate 1 bis 8 bzw. V1, 15 g Kokosfettalkohol+2EO-sulfat-Natriumsalz, 3 g Dimethylpolysiloxan, 5 g Kokosalkylglucosid und 1,5 g eines Esterquats (Wasser ad 100 Gew.-%) enthielten. Die Feinteiligkeit der Perlglanzkristalle in den Haarshampoos wurde unter dem Mikroskop visuell auf einer Skala von 1 = sehr feine Kristalle bis 5 = grobe Kristalle beurteilt. Die Beurteilung des Perlglanzes erfolgte ebenfalls auf einer Skala von 1 = brillant bis 5 = stumpf; die Trübung wurde visuell bestimmt und mit (+) = trüb oder (-) = trübungsfrei beurteilt. Die Zusammensetzungen und Ergebnisse sind in Tabelle 1 zusammengefaßt; alle Mengenangaben verstehen sich als Gew.-%

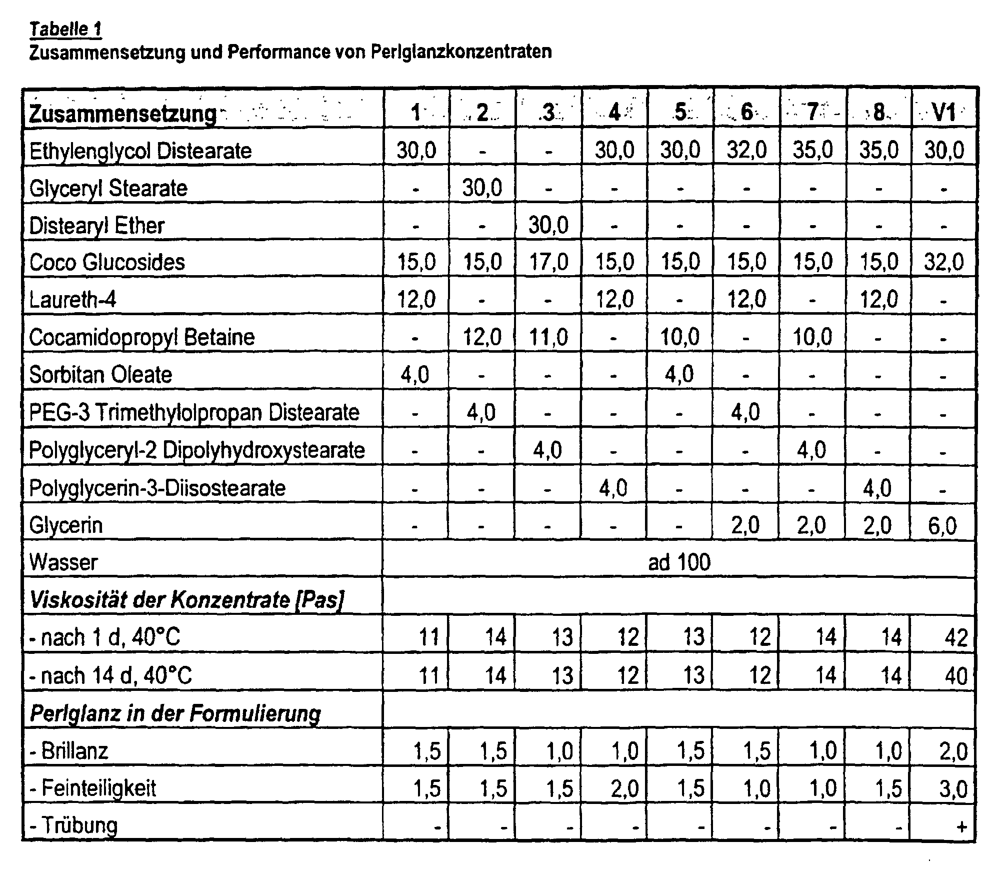

## Patentansprüche

1. Hochkonzentriert fließfähige Perlglanzkonzentrate, enthaltend
(a) 25 bis 45 Gew.-% Perlglanzwachse,
(b) 25 bis 40 Gew.-% nichtionische, amphotere, zwitterionische und/oder kationische Emulgatoren und
(c) 0,5 bis 15 Gew.-% Polyolester
mit den Maßgaben, daß sich die Mengenangaben mit Wasser und gegebenenfalls weiteren Hilfsund Zusatzstoffen zu 100 Gew.-% ergänzen und die Summe der Komponenten (a), (b) und (c) mindestens 55 Gew.-% beträgt.

2. Konzentrate nach Anspruch 1, **dadurch gekennzeichnet, daß** sie als Komponente (a) Perlglanzwachse enthalten, die ausgewählt sind aus der Gruppe, die gebildet wird von Alkylenglycolestern, Fettsäurealkanolamiden, Partialglyceriden, Estern von mehrwertigen, gegebenenfalls hydroxysubstituierte Carbonsäuren, Fettalkoholen, Fettsäuren, Fettketonen, Fettaldehyden, Fettethern, Fettcarbonaten, Ringöffnungsprodukten von Olefinepoxiden sowie deren Mischungen.

3. Konzentrate nach den Ansprüchen 1 und/oder 2, **dadurch gekennzeichnet, daß** sie als Komponente (b) nichtionische Tenside enthalten, die ausgewählt sind aus der Gruppe, die gebildet wird von Anlagerungsprodukten von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest; Alkylmono- und -oligoglycosiden mit 8 bis 22 Kohlenstoffatomen im Alkylrest und deren ethoxylierte Analoga; Anlagerungsprodukte von 1 bis 15 bzw. 15 bis 60 Mol Ethylenoxid an Ricinusöl und/oder gehärtetes Ricinusöl; Mono-, Di- und Trialkylphosphaten sowie Mono-, Di- und/oder Tri-PEG-alkylphosphaten und deren Salze; Wollwachsalkoholen; Polysiloxan-Polyalkyl-Polyether-Copolymeren bzw. entsprechenden Derivaten; Polyalkylenglycolen sowie Glycerincarbonat.

4. Konzentrate nach mindestens einem der Ansprüche 1 bis 3, **dadurch gekennzeichnet, daß** sie als Komponente (b) Cocamidopropylbetaine und/oder Esterquats enthalten.

5. Konzentrate nach mindestens einem der Ansprüche 1 bis 4, **dadurch gekennzeichnet, daß** sie als Komponente (c) Polyolester enthalten, die ausgewählt sind aus der Gruppe, die gebildet wird von Partialestern von Glycerin und/oder Sorbitan mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid; Partialestern von Polyglycerin, Polyethylenglycol, Trimethylolpropan, Pentaerythrit, Alkylglucosiden sowie Polyglucosiden mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid; Mischestern aus Pentaerythrit, Fettsäuren, Citronensäure und Fettalkohol und/oder Mischester von Fettsäuren mit 6 bis 22 Kohlenstoffatomen, Methylglucose und Polyole sowie deren Gemischen.

6. Konzentrate nach mindestens einem der Ansprüche 1 bis 5, **dadurch gekennzeichnet, daß** sie als fakultative Komponente (d) Polyole enthalten.

7. Konzentrate nach Anspruch 6, **dadurch gekennzeichnet, daß** sie als Polyole Glycerin und/oder Ethylenglycol enthalten.

8. Konzentrate nach den Ansprüchen 5 und/oder 6, **dadurch gekennzeichnet, daß** sie 0,1 bis 15 Gew.-% - bezogen auf die Mittel - Polyole enthalten.

9. Verfahren zur Herstellung zur Herstellung von Perlglanzkonzentraten nach Anspruch 1, **dadurch gekennzeichnet, daß** man eine Mischung aus den Komponenten (a), (b) (c) und gegebenenfalls (d) herstellt, auf eine Temperatur erwärmt, die 1 bis 30°C oberhalb des Schmelzpunktes der Mischung liegt, mit der erforderlichen Menge Wasser etwa der gleichen Temperatur mischt und anschließend auf Raumtemperatur abkühlt.

10. Verwendung von Polyolestern als Viskositätsregulatoren zur Herstellung von Perlglanzkonzentraten mit Aktivsubstanzgehalten von mindestens 55 Gew.-%.

## Claims

1. Highly concentrated flowable pearlizing concentrates containing
(a) 25 to 45% by weight of pearlizing waxes,
(b) 25 to 40% by weight of nonionic, amphoteric, zwitterionic and/or cationic emulsifiers and
(c) 0.5 to 15% by weight of polyol esters,
with the provisos that the quantities shown add up to 100% by weight with water and optionally other auxiliaries and additives and the sum of components (a), (b) and (c) is at least 55% by weight.

2. Concentrates as claimed in claim 1, **characterized in that** they contain as component (a) pearlizing waxes selected from the group consisting of alkylene glycol esters, fatty acid alkanolamides, partial glycerides, esters of polybasic, optionally hydroxysubstituted carboxylic acids, fatty alcohols, fatty acids, fatty ketones, fatty aldehydes, fatty ethers, fatty carbonates, ring opening products of olefin epoxides and mixtures thereof.

3. Concentrates as claimed in claims 1 and/or 2, **characterized in that** they contain as component (b) nonionic surfactants selected from the group consisting of products of the addition of 2 to 30 mol ethylene oxide and/or 0 to 5 mol propylene oxide onto linear fatty alcohols containing 8 to 22 carbon atoms, onto fatty acids containing 12 to 22 carbon atoms, onto alkylphenols containing 8 to 15 carbon atoms in the alkyl group and onto alkylamines containing 8 to 22 carbon atoms in the alkyl group; alkyl mono- and oligoglycosides containing 8 to 22 carbon atoms in the alkyl group and ethoxylated analogs thereof; products of the addition of 1 to 15 or 15 to 60 mol ethylene oxide onto castor oil and/or hydrogenated castor oil; mono-, di- and trialkyl phosphates and mono-, di- and/or tri-PEG-alkyl phosphates and salts thereof; wool wax alcohols; polysiloxane/polyalkyl polyether copolymers and corresponding derivatives; polyalkylene glycols and glycerol carbonate.

4. Concentrates as claimed in at least one of claims 1 to 3,
**characterized in that** they contain Cocamidopropyl Betaine and/or esterquats as component (c).

5. Concentrates as claimed in at least one of claims 1 to 4, **characterized in that** they contain as component (c) polyolesters selected from the group consisting of partial esters of glycerol and/or sorbitan with saturated and/or unsaturated, linear or branched fatty acids containing 12 to 22 carbon atoms and/or hydroxycarboxylic acids containing 3 to 18 carbon atoms and adducts thereof with 1 to 30 mol ethylene oxide; partial esters of polyglycerol, polyethylene glycol, trimethylolpropane, pentaerythritol, alkyl glucosides and polyglucosides with saturated and/or unsaturated, linear or branched fatty acids containing 12 to 22 carbon atoms and/or hydroxycarboxylic acids containing 3 to 18 carbon atoms and adducts thereof with 1 to 30 mol ethylene oxide; mixed esters of pentaerythritol, fatty acids, citric acid and fatty alcohol and/or mixed esters of fatty acids containing 6 to 22 carbon atoms, methyl glucose and polyols and mixtures thereof.

6. Concentrates as claimed in at least one of claims 1 to 5, **characterized in that** they contain polyols as optional component (d).

7. Concentrates as claimed in claim 6, **characterized in that** they contain glycerol and/or ethylene glycol as polyols.

8. Concentrates as claimed in claims 5 and/or 6, **characterized in that** they contain 0.1 to 15% by weight, based on the preparation, of polyols.

9. A process for the production of the pearlizing concentrates claimed in claim 1, **characterized in that** a mixture of components (a), (b), (c) and optionally (d) is prepared, heated to a temperature 1 to 30°C above the melting point of the mixture, mixed with the necessary quantity of water at about the same temperature and then cooled to room temperature.

10. The use of polyol esters as viscosity adjusters for the production of pearlizing concentrates with active substance contents of at least 55% by weight.

## Revendications

1. Concentrés nacrés fluides hautement concentrés contenant
(a) 25 à 45 % en poids de cires nacrées,
(b) 25 à 40 % en poids d'émulsifiants non ioniques, amphotères, zwitterioniques et/ou cationiques et
(c) 0,5 à 15 % en poids d'ester de polyol
étant précisé que les quantités indiquées sont complétées avec de l'eau et, éventuellement, d'autres adjuvants et additifs, jusqu'à 100 % en poids et que le total des composants (a), (b) et (c) représente au moins 55 % en poids.

2. Concentrés selon la revendication 1,
**caractérisés en ce que**
comme composant (a), ils contiennent, des cires nacrées choisies dans le groupe constitué d'esters d'alkylène glycol, d'alcanolamides d'acides gras, de glycérides partiels, d'esters d'acides carboxyliques polyvalents, le cas échéant substitués par un groupe hydroxy, d'alcools gras, d'acides gras, de cétones grasses, d'aldéhydes gras, d'éthers gras, de carbonates gras, de produits d'ouverture de cycle d'époxydes d'oléfines, ainsi que de leurs mélanges.

3. Concentrés selon les revendications 1 et/ou 2,
**caractérisés en ce que**
comme composant (b) ils contiennent, des tensioactifs non ioniques choisis dans le groupe constitué de produits d'addition de 2 à 30 moles d'oxyde d'éthylène et/ou de 0 à 5 moles d'oxyde de propylène sur des alcools gras linéaires comportant 8 à 22 atomes de C, sur des acides gras comportant 12 à 22 atomes de C, sur des alkylphénols comportant 8 à 15 atomes de C dans le groupe alkyle, ainsi que sur des alkylamines comportant 8 à 22 atomes de carbone dans le radical alkyle ; de mono et oligoglycosides d'alkyle comportant 8 à 22 atomes de carbone dans le radical alkyle et de leurs analogues éthoxylés ; de produits d'addition de 1 à 15 ou de 15 à 60 moles d'oxyde d'éthylène sur de l'huile de ricin et/ou de l'huile de ricin durcie ; de phosphates de mono-, di- et trialkyle ainsi que de phosphates de mono-, di- et/ou tri-PEG-alkyle et de leurs sels ; d'alcools de lanoline ; de copolymères polysiloxane, -polyalkyle-polyéther ou de dérivés correspondants ; des polyalkylène glycols ainsi que du carbonate de glycérol.

4. Concentrés selon au moins l'une des revendications 1 à 3,
**caractérisés en ce que**
comme composant (b), ils contiennent des cocamidopropylbétaïnes et/ou des ammonium quaternaires estérifiés.

5. Concentrés selon au moins l'une des revendications 1 à 4,
**caractérisés en ce que**
comme composant (c), ils contiennent, des esters de polyol choisis dans le groupe constitué d'esters partiels de glycérol et/ou de sorbitane et d'acides gras saturés et/ou insaturés, linéaires ou ramifiés comportant 12 à 22 atomes de carbone et/ou d'acides hydroxycarboxyliques, comportant 3 à 18 atomes de carbone ainsi que leurs produits d'addition avec 1 à 30 moles d'oxyde d'éthylène ; d'esters partiels de polyglycérol, de polyéthylène glycol, de triméthylol propane, de pentaérythrite, de glucosides d'alkyle et de polyglucosides et d'acides gras saturés et/ou insaturés, linéaires ou ramifiés comportant 12 à 22 atomes de carbone et/ou d'acides hydroxycarboxyliques comportant 3 à 18 atomes de carbone ainsi que de leurs produits d'addition avec 1 à 30 moles d'oxyde d'éthylène ; d'esters mixtes de pentaérythrite, d'acides gras, d'acide citrique et d'alcool gras et/ou d'esters mixtes d'acides gras comportant 6 à 22 atomes de carbone, de méthylglucose et de polyols ainsi que de leurs mélanges.

6. Concentrés selon au moins l'une des revendications 1 à 5,
**caractérisés en ce qu'**
ils contiennent des polyols en tant que composant (d) facultatif.

7. Concentrés selon la revendication 6,
**caractérisés en ce que**
comme polycols ils contiennent du glycérol et/ou de l'éthylène glycol.

8. Concentrés selon les revendications 5 et/ou 6,
**caractérisés en ce qu'**
ils contiennent 0,1 à 15 % en poids de polyols par rapport au milieu.

9. Procédé de préparation de concentrés nacrés selon la revendication 1,
**caractérisé en ce qu'**
on prépare un mélange constitué des composants (a), (b), (c) et, le cas échéant, (d), on le chauffe à une température supérieure de 1 à 30°C à son point de fusion, on le mélange avec la quantité d'eau nécessaire approximativement à la même température puis on le refroidit à la température ambiante.

10. Utilisation d'esters de polyols en tant que régulateurs de la viscosité, pour la préparation de concentrés nacrés contenant au moins 55 % en poids de substances actives.
